# EUROPEAN PATENT SPECIFICATION

(11) **EP 2 177 169 B1**
(45) Date of publication and mention of the grant of the patent: **04.05.2016**
(21) Application number: 08425672.6
(22) Date of filing: 16.10.2008
(51) Int. Cl.: A61B 17/32, A61B 17/22, A61B 17/00

(54) **Vibrating device for the treatment of adipose tissue**
Vibrationsvorrichtung zur Behandlung von adipösem Gewebe
Dispositif vibrant pour le traitement de tissu adipeux

(43) Date of publication of application: 21.04.2010
(73) Proprietor: Lain Electronic S.r.L., 20011 Corbetta MI (IT)
(72) Inventor: Marzorati, Armando, 20010 Santo Stefano Ticino (Milano) (IT)
(74) Representative: Lunati, Valerio

(56) References cited:
- US-A- 4 922 902
- US-A- 5 419 761
- US-A1- 2002 049 551
- US-A1- 2002 124 617
- US-A1- 2004 162 546
- US-A1- 2006 100 547

## Description

The present invention relates to a vibrating device for the treatment of adlpose tissue of the type specified in the preamble of claim 1.

There are currently known different devices for surgical treatment of adipose tissue, in particular to perform liposuction treatments, which consist in the surgical removal of portions of fat from the human body.

These devices are substantially realized by a suction cannula which is introduced into the areas to be treated.

The cannula aspirates the adipose cells, thereby eliminating the layer of fat. This treatment is very traumatic for the human body and very dangerous if not performed correctly.

In fact, forced aspiration of the adipose cells implies undesirable aspiration of non-adipose cells and, for example, of capillaries, muscle cells and the like. Attempts have been made to remedy this drawback by means of specific devices and techniques which make use of the cavitation phenomenon. Devices using cavitation phenomenon are described for example in patent documents US-A-2004/0162546 and US-A-5419761.

This technique consists in introducing a vibrating probe into the human body, and in particular in the area to be treated.

Due to its motion, said vibrating probe produces very high variations in pressure.

These variations in pressure cause, inside the aqueous environment in which the lipid tissue to be removed Is immersed, gas microbubbles due to local ebullition of the liquid, which is locally at a lower pressure than the vapour pressure.

The bubbles formed are unstable and rapidly implode causing very high min-uscule changes in pressure which cause damage to the adipose cells.

The damaged adipose cells, substantially in liquid form, can then be easily removed by low pressure suction or the like.

This low pressure suction does not cause damage to the surrounding tissues.

However, also in this second case, the aforesaid prior art presents some important drawbacks and risks for the patient's health.

In fact, although the suction of damaged tissues is not harmful, the cavitation phenomenon thus applied can cause damage not only to adipose tissue but also to important functional tissue such as cartilage, muscle tissue and the like.

In particular, if the vibrating probe is positioned and maintained in proximity of a cartilage or another non-adipose tissue, this is damaged to the same extent as the adipose tissue.

Moreover, said vibrating probes have very low efficiency and dissipate a large amounts of thermal energy.

Another drawback of prior art is realized by the fact that, due to the dimensions and power and to the secondary effects of cannulas, it is not possible to use them to treat adipose tissue located in delicate areas of the human body.

Therefore, it is not possible to treat adipose tissue on the face and neck, in particular in proximity of the chin.

This drawback can lead to overheating of the vibrating probe and burning of the patient undergoing liposuction treatment.

In this situation, the technical aim of the present invention is to devise a vibrating device for the treatment of adipose tissue capable of substantially overcoming the aforesaid drawbacks.

Within said technical aim, an important object of the invention is to obtain a vibrating device for the treatment of adipose tissue which does not damage non-adipose tissue or in any case tissue that is not to be intentionally removed.

Another important object of the invention is to produce a vibrating device for the treatment of adipose tissue which has high efficiency and does not burn the patient.

A further technical aim of the present invention is to obtain a vibrating device that allows treatment of adipose tissue in delicate areas of the human body, such as the neck, chin and face.

The technical aim and the objects specified are achieved by a vibrating device for the treatment of adipose tissue as claimed in the appended claim 1. Preferred executions are specified in the sub-claims.

The features and advantages of the invention are better explained below in the detailed description of a preferred embodiment of the invention, with reference to the attached drawings, wherein:

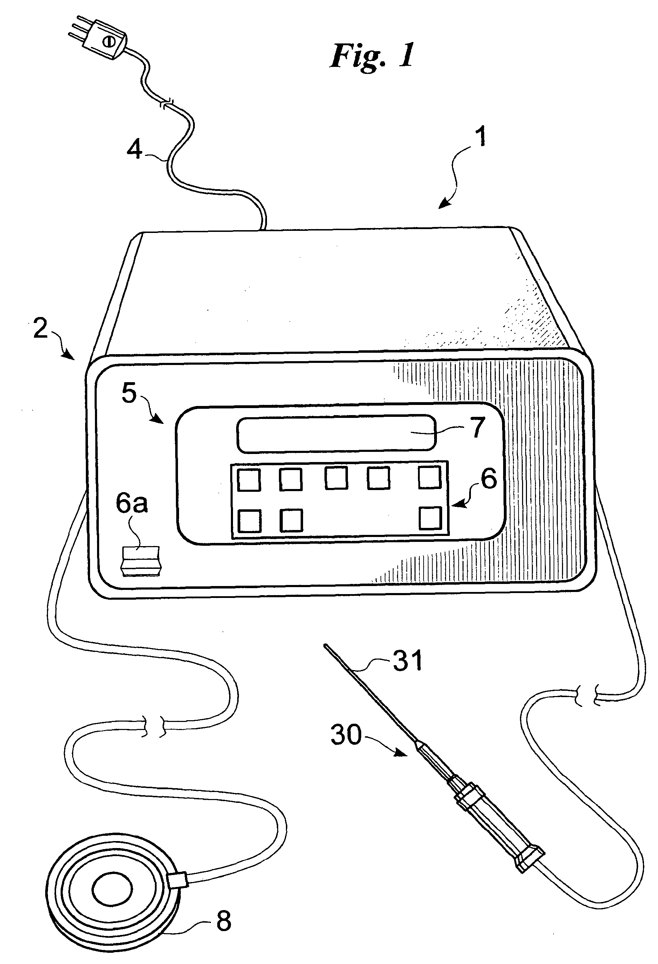

**Fig. 1** shows the external appearance of the device according to the invention;
**Fig. 2** shows an operating diagram of the device according to the invention;
**Fig. 3** shows a first example of embodiment of a part of the device according to the invention in an exploded view;
**Fig. 4** presents a side view in elevation of a part of the device according to the invention;
**Fig. 5** shows a sectional view along the median line of a part of the device according to the invention; and
**Fig. 6** shows a three-dimensional axonometric view of the part of Fig. 5.
With reference to the Figures, the vibrating device according to the invention, is indicate as a whole with the number **1**.

It principally comprises a generator apparatus **2**, suitable to generate, or more simply to emit, alternating electric current, and one or more probes **30**, electrically connected to the generator apparatus **2** and suitable to cause cavitation phenomena when in contact with an aqueous liquid, when activated by the alternating electric current coming from the generator apparatus 2.

The probes 30 comprise a tip **31**, suitable to be introduced into the human body in areas comprising adipose tissue that are to be treated and a plurality of piezoelectric units **32**, suitable to transform the alternating electric current into mechanical vibrations.

More in particular, the piezoelectric units 32 are realized by ceramic material or the like capable of transforming electrical energy into mechanical energy and vice versa.

The electrical and mechanical properties in the piezoelectric units 32 are strictly correlated, for example the electrical power received and the mechanical power emitted are directly proportional, and therefore it is possible to determine the latter by measuring the former.

In particular, when the piezoelectric units 32 are energized with alternating electric current at a specific frequency, they generate mechanical vibrations of the same frequency and with a power directly proportional to the absorbed power.

As it is known, said mechanical and electrical power is inversely proportional to the mechanical and electrical impedance. Therefore, these last two parameters are also directly proportional.

Said impedance varies as a function of the vibrating system, in the present case realized by the piezoelectric units 32, by the probes 30 and by the environment surrounding them, and of the vibration frequency.

Moreover, said probes 30 present at least one resonance frequency, in proximity of which the impedance assumes particularly low values and therefore the power assumes a very high value.

The probes 30 are supplied by the generator apparatus 2 with electrical current having frequencies in close proximity of, or coinciding with, the resonance frequency of the system composed of the probe 30 and of the surrounding environment.

Therefore, they produce the high powers necessary to produce the cavitation phenomenon, with low energy consumption.

However, said resonance frequency, which as specified varies as a function both of the properties of the probe 30 and of the environment surrounding it, in particular according to the environment surrounding the tip 31, does not remain constant during treatment but varies as a function of the environment in which the tip 31 is immersed.

In particular, the probe 30 is initially supplied by the generator apparatus 2 with alternating electric current having frequencies in close proximity of the resonance frequency of the probe immersed in specific environments.

The term specific environments indicates the environments to be treated, in particular liquid environments having density similar to that of water, or the adipose accumulations.

Therefore, the device 1 is capable of causing cavitation only in said specific environments, as otherwise the impedance of the system increases excessively and the vibration frequencies of the probe 30 no longer correspond to the resonance frequencies. Therefore, in this latter case, the probe 30 is not capable of producing cavitation.

For example, the probe 30 is not capable of causing cavitation if it comes into contact with muscle, bone or cartilage.

The generator apparatus 2 also comprises a control system 3 of the electrical power transmitted by the generator 2 to the probe 30 at various frequencies.

This control system 3 is capable of detecting the electrical power transmitted at different frequencies, proportional to the mechanical power of the vibrations.

Therefore, it is capable of detecting whether or not the probe 30, and in particular the tip 31, has come into contact with one of said specific environments, by detecting the electrical impedance of the system.

Consequently, if the probe 30 comes into contact with body fluids denser than water, for example realized by the adipose tissues or the like, the control system 3 is preferably capable of distinguishing the low variation of impedance and of regulating the emission frequency of the electrical current so as to supply the probe 30 with alternating electric current having frequencies in close proximity of, or coinciding with, the new resonance frequency of the system.

In yet other cases variation of the resonance frequency can be due to variation of the probe 30, in particular to a crack or chip or the like that has formed in the tip 31 which is always subjected to high stresses.

Also in this case the resonance frequency varies considerably and the operator is therefore able, when indicated by the device 1, to detect the malfunction and to replace the tip before this can cause damage or break inside the human body.

In detail, the device 1 is connected to the mains power supply through specific connection means 4, substantially realized by an electrical cable and a plug.

The generator apparatus 2 is controllable through the control system 3, included in the apparatus 2.

The control system 3 also comprises control means **5**, appropriately actuatable manually and comprising, for example, a keypad **6**, comprising a switch **6a** for switching on or off the power from the mains power supply, and a display **7** for viewing the selected parameters.

The control means 5 also preferably comprise a push button switch **8** appropriately actuatable with a pedal and connected by means of a specific connection to the apparatus 2. This push button switch is suitable to activate the probe 30.

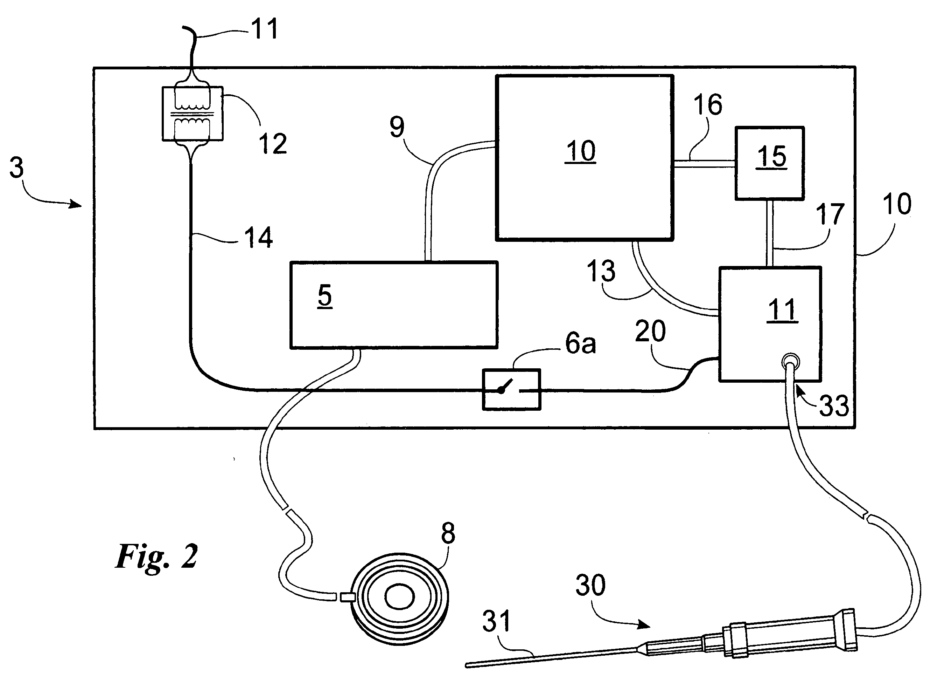

The control means 5 are connected, by means of appropriate first connection means **9**, to a control and processing unit **10** (Fig. 2).

The control system 3 also comprises a controller board **15** and a power circuit **11**.

The control and processing unit 10 comprises a microcomputer and connected memories for data processing. It can be programmed through the control means 5.

The power supply of the control and processing unit 10 is preferably supplied by the power circuit 11 through second connection means **13**.

Moreover, the controller board **15**, suitable to perform interface functions between the control and processing unit 10 and the power circuit 11, is interposed between the control and processing unit 10 and the power circuit 11.

It is connected to the control and processing circuit 10 and to the power circuit 11 respectively by means of third and fourth connection means **16** and **17**.

The controller board 15 amplifies and isolates the control signals of the power circuit 11 and processes the logic and analogue signals to make them compatible with the voltage levels of the microcomputer.

Moreover, the controller board 15 receives from the power circuit 11 a signal proportional to the current and to the voltage present in the probe 30, the values of which are multiplied and transmitted to the control and processing unit 10.

Moreover, the controller board 15 comprises a programmable frequency generator, appropriately of the type known as "programmable logic device (PLD)".

The apparatus 2 appropriately also comprises an isolation transformer **12** directly connected to the mains power supply and suitable to isolate it from the power circuit 11 and/or from the control and processing unit 10 and from the controller board 15. Consequently, the vibrations and the fluctuations in the mains power supply do not influence the circuit 11, the board 15 or the unit 10.

The power circuit 11 is instead preferably supplied by an isolation transformer 12, by means of fifth connection means **14**; it also preferably comprises a half-bridge resonant inverter suitable to generate sinusoidal waves.

This inverter thus supplies the electrical power required to activate the probes 30. The electrical power is propagated through releasable electrical connection means **33** to the transducer 30.

The releasable electrical connection means 33 are substantially realized by an electrical cable and a movable connection such as a plug and an electrical socket and have a length or capacity that allows easy handling of the probe 30.

The probes 30 thus transform the alternating electric current coming from the power circuit 11 into mechanical vibrations of the same frequencies.

As specified, the probes 30 present specific resonance frequencies, and are realized so as to obtain, in water, resonance frequencies at frequency intervals between 15 kHz and 50 kHz, more in particular at 20 kHz and at 40 kHz or also even only at 40 kHz. In fact, as described above, when cavitation is produced in these vibration intervals the positive effects of removal of adipose tissue linked to cavitation are maximized.

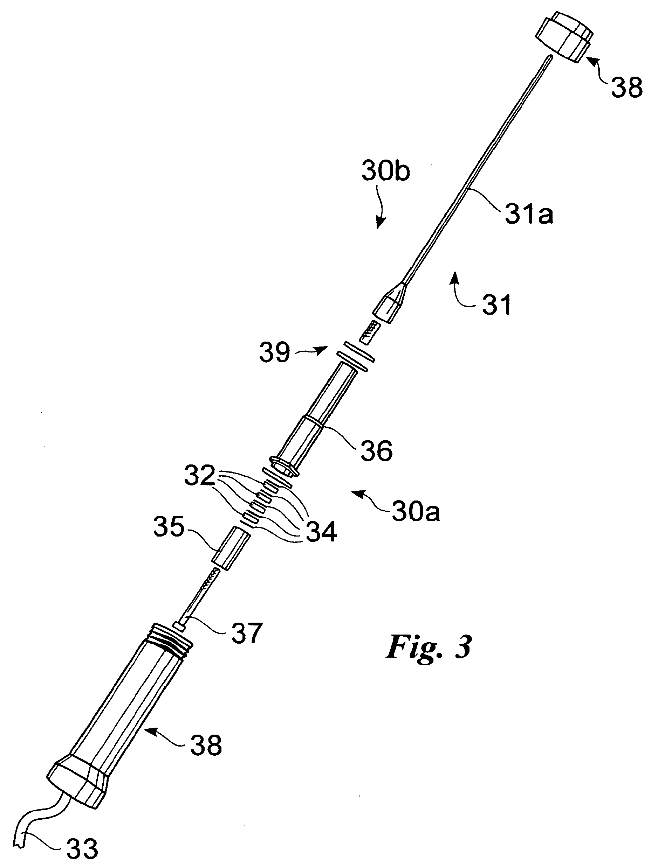

A first probe **30b** is shown in Fig. 3.

It comprises a handpiece **30a** including the piezoelectric units 32.

These piezoelectric units 32 are made of piezoelectric ceramic material preferably realized by a lead-titanate intermetallic ceramic. They are preferably disk-shaped and are also preferably positioned stacked and electrically connected to the releasable electrical connection means 33 through appropriate conductor elements **34**.

The conductor elements 34, which are electrically connected to the releasable electrical connection means 33, are also appropriately disk-shaped and produced in Copper alloy or other conductor metals and interposed between said stacked piezoelectric units 32.

The handpiece 30a also preferably comprises a first and a second locking body, cylindrical in shape and produced in metal materials, preferably respectively in steel and in titanium alloy.

The locking bodies **35** and **36** are preferably stacked at the two ends of the piezoelectric units 33 and of the conductor elements 34, and enclose these through specific clamping means **37**, appropriately realized by a socket-head screw engageable with the second locking body 36.

The piezoelectric units 33, the locking bodies 35 and 36 and the clamping means 37 have the purpose of allowing correct channelling of the mechanical vibrations and transmission thereof to the tip 31, connectable to the handpiece 30a, as described below.

To allow clamping of the locking bodies 35 and 36, of the piezoelectric units 33 and of the conductor elements 34, these named elements present a central hole through which the clamping means 37 pass.

In particular, the clamping means 37 engage on the first locking body 35, appropriately through the head of the means 37 of greater dimensions than those of the hole produced in said locking body 35, and on the second locking body 36 presenting a threaded blind hole. The clamping means 37 thus sandwich the various elements of which the transducer is composed. Moreover, the handpiece 30a comprises a cylindrical retaining body **38**, preferably produced in aluminium and including the locking bodies 35 and 36, the piezoelectric units 33, the clamping means 37 and the other elements described.

It is suitable to be manually gripped and handled. In particular, two different handpieces 30a are provided, suitable to support tips 31 of different sizes. Finally, the handpiece 30a comprises releasable coupling means **39**, suitable to allow fastening of the tip 31 to the handpiece and replacement thereof. These are appropriately realized by a screw and by engaging means for a clamping key present at the base of the tip 31.

In particular, on the first probe 30a, suitable to treat adipose tissue present on the flanks and abdomen, the tip 31 is realized by a long tip **31a** realized by a grade 5 titanium alloy needle. In particular, three long tips 31a are provided, having lengths between 10 cm and 40 cm and more precisely 18.5 cm, 22 cm and 32 cm and diameters between 0.5 mm and 5 mm and more precisely 2 mm and 2.5 mm.

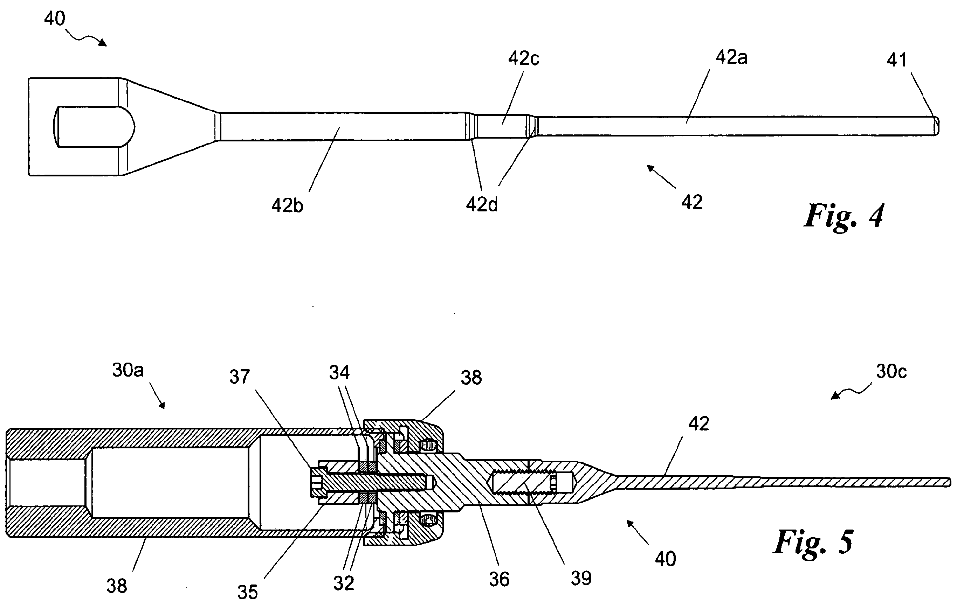

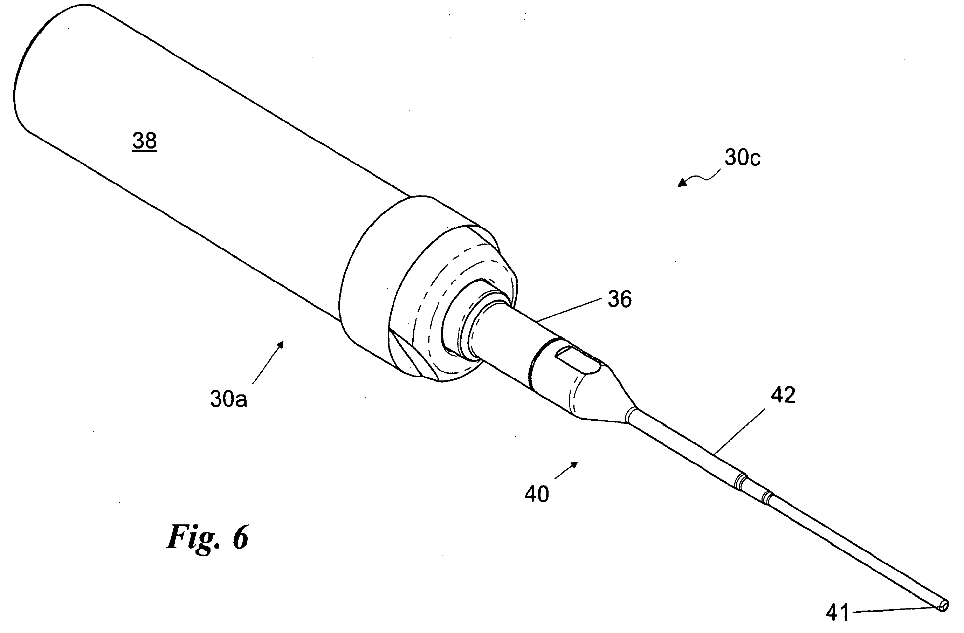

A second probe **30c** is shown in the details in Figs. 4-6.

This probe comprises a tip 31 realized by a fine tip **40**, shown in scale in Fig. 4, having a diameter in the terminal part **41** between 1.5 mm and 2.5 mm and in particular in proximity of 2.1 mm.

Said probe is thus suitable to be used in delicate areas of the human body, such as the neck, chin and face.

In particular, the fine tip 40 comprises a tubular portion **42** having a length between 7 cm and 10 cm and more in particular between 80 mm and 85 mm. Moreover, this tubular portion 42 comprises at least two cylindrical portions having different diameters.

In detail, a first cylindrical portion **42a** comprising the terminal part 41 is present, having a length between 3 cm and 6 cm, and more in particular between 45 mm and 50 mm, and a diameter equal to said diameter of the terminal part 41.

A second cylindrical portion **42b** is also present, comprising the base of the fine tip 40, having a length between 2 cm and 4 cm, and more in particular between 25 mm and 30 mm, and a diameter between 2.5 mm and 3.5 mm and in particular in proximity of 3.1 mm.

A third cylindrical portion **42c** is also preferably present, interposed between the first and second cylindrical portion 42a and 42b and having a length between 0.5 cm and 1.5 cm, and more in particular between 25 mm and 30 mm, and a diameter between 2.5 mm and 3.5 mm and in particular in proximity of 3.1 mm.

The cylindrical portions 42a-42c are reciprocally connected by means of connectors **42d** having lengths of approximately one millimetre.

Also the tip 40 is preferably made of grade 5 titanium.

The remaining elements of the second probe 30c have functions, materials and shapes analogous to the elements of which the first probe 30b is composed and are shown in scale and in detail in the section of Fig. 4 and in Fig. 5.

The second probe 30c has a resonance frequency in particular at 40 kHz. Operation of the vibrating device 1, the structure of which is described above, is as follows.

The operator, who performs the treatment on the patient treated, first abundantly infiltrates the adipose tissue with saline solution, to achieve an intense cavitation effect, which is better produced in a liquid medium, causing extremely effective mechanical microtraumas.

The operator who performs the treatment grips the probe 30 and introduces the tip 31 into the treated portion of the body of the patient being treated, through a small opening made in the skin.

Vibration of the tip 31 then causes said cavitation phenomenon, at the frequencies indicated.

The cavitation phenomenon is caused at the resonance frequency of the probe 30 and surrounding environment system, so that low quantities of energy are sufficient to produce a high power.

The adipose tissue is therefore damaged by implosions of the cavitation bubbles.

Cell fragmentation caused by the implosions leads to diffusion of the various components of the cytoplasm in the interstitial spaces and mixing of the products of cell lysis with the solution employed for initial preparation of the tissue.

The lipid emulsion that is thus formed can be easily aspirated with a cannula connected to an aspirator or removed simply by squeezing the part.

If the operator were to unintentionally position the tip 31 in contact with a muscle, bone or cartilage, the impedance of the probe 30 and surrounding environment system would increase considerably and said cavitation phenomenon would not take place, and consequently said parts would not be damaged.

Moreover, in this case the control and processing unit 10, warned by the controller board 15, detects the low power emitted by the probe 30 and consequently detects any contact of the tip with a portion of the body not to be removed.

The apparatus 2 indicates this fault to the operator by means of the display 7 or the like. The operator can therefore move the probe 30 and resume the operation.

Differently, if the tip 31 comes into direct contact with the adipose tissue or with liquids with different density to water, the impedance of the probe 30 and surrounding environment system increases slightly, decreasing the efficiency of the device.

In this case the control and processing unit 10, warned by the controller board 15, detects the lower power emitted by the probe 30 and the different powers emitted by the probe 30 at different vibration frequencies. Consequently, the control and processing unit 10 regulates the controller board 15, which transmits the frequency corresponding to the resonance frequency to the power circuit 11.

In this way the probe 30 always vibrates at resonance frequency and therefore consumption is always minimum.

Finally, if the tip 31 were to chip or break, the impedance of the probe 30 and surrounding environment system would increase considerably and the control and processing unit 10 would detect the low power emitted by the probe 30 and therefore any breakage.

The invention achieves important advantages.

In fact, the device 1 does not cause damage to non-adipose tissue or in any case to tissue that is not to be removed, due to the fact that it operates at resonance frequency.

Moreover, the device has very high efficiency. In fact, it can be activated with a power of less than one fifth of the power required to activate prior art devices.

Consequently, the device 1 produces less thermal energy and does not risk burning the patient, unlike prior art devices which must be appropriately shielded by insulating material.

A further advantage of the device 1 is given by the geometry and by the materials of the second probe 30c.

In fact, this second probe 30c makes it possible to treat adipose tissue in delicate areas of the human body, such as the neck, chin and face.

The present advantage is due in particular to the thickness and structure of the fine tip 40, which in view of its small diameter can be used in delicate areas. The particular geometry of the fine tip 40, and of the second probe 30c in general, also allows resonance to be obtained at 40 kHz. In fact, this frequency is suitable to treat said delicate areas.

## Claims

1. Vibrating device for the treatment of adipose tissues comprising: at least one generator apparatus (2) of alternating electric current, at least one probe (30) connected to said generator apparatus (2) and including: at least one piezoelectric element (32), suitable to transform said alternating electric current into mechanical vibrations, and at least one tip (31, 40), suitable to be introduced into said adipose tissue, to transmit said mechanical vibrations and to cause cavitation inside said adipose tissue, said probe (30) presenting at least one resonance frequency variable according to said probe (30) and to the environment surrounding said tip (31), a control system (3) suitable to verify the resonance frequency of said probe (30) immersed in the surrounding environment, and to regulate the emission frequency of the electrical current so as to supply to said probe (30) frequencies prevalently in proximity of the resonance frequency of said probe (30) when said tip is immersed in specific environments to be treated, so as to cause cavitation only if said tip (31) is immersed in said specific environments, wherein said control system (3) is suitable to indicate important variations of the resonance frequency of said probe (30) and to avoid damaging of non-adipose tissues by not regulating, in this cases, the emission frequency of the electrical current.

2. Device according to Claim 1, suitable to cause cavitation only if said tip (31) is immersed in water or in a fluid of similar density.

3. Device according to Claim 2, wherein said probe (30) immersed in water presents a resonance frequency in proximity of 20 kHz.

4. Device according to Claim 2 or 3, wherein said probe (30) immersed in water presents a resonance frequency in proximity of 40 kHz.

5. Device according to one or more of the preceding Claims, wherein said probe comprises a handpiece (30a) connectable to said tip (31) and comprising a plurality of piezoelectric elements (32) connected to conductor elements (34) and two locking bodies (35) and (36) suitable to sandwich said piezoelectric elements (32) through specific clamping means (37).

6. Device according to one or more of the preceding Claims, wherein said tip (31) is realized by a needle having a length between 10 cm and 40 cm and a diameter between 0.5 m and 5 mm.

7. Device according to one or more of the preceding Claims, wherein said tip (31) is made of grade 5 titanium.

8. Device according to one or more of the preceding Claims, wherein said tip (31) is realized by a long tip (31a) realized by a needle having a length between 10 cm and 40 cm and a diameter between 0.5 mm and 5 mm.

9. Device according to one or more of the preceding Claims, wherein said tip (31) is realized by a fine tip (40) comprising a tubular portion (42) having a length between 7 cm and 10 cm and a diameter of the terminal part thereof (41) between 1.5 mm and 2.5 mm

10. Device according to Claim 10, wherein said tubular portion (42) comprises at least a first and a second cylindrical portion (42a, 42b) having different diameters.

11. Device according to Claim 10, wherein said first cylindrical portion (42a) comprises said terminal part (41) and has a length between 3 cm and 6 cm and a diameter equivalent to the diameter of said terminal part (41).

12. Device according to Claim 10 or 11, wherein said second cylindrical portion (42b) comprises the base of said fine tip (40) and has a length between 2 cm and 4 cm and a diameter between 2.5 mm and 3.5 mm.

13. Device according to one or more of Claims 10-12, wherein said tubular portion (42) comprises a third cylindrical portion (42c), interposed between said first and said second cylindrical portion (42a, 42b) and having a length between 0.5 cm and 1.5 cm and a diameter between 2.5 mm and 3.5 mm.

## Patentansprüche

1. Vibrationsvorrichtung zur Behandlung von adipösem Gewebe, die Folgendes umfasst: wenigstens einen Wechselstromgenerator (2), wenigstens eine an den genannten Wechselstromgenerator (2) angeschlossene Sonde (30), die mindestens ein piezoelektrisches Element (32) enthält, das geeignet ist, den genannten Wechselstrom in mechanische Vibrationen umzuwandeln, und mindestens eine Spitze (31, 40), die geeignet ist, in das genannte adipöse Gewebe eingeführt zu werden, um die genannten mechanischen Vibrationen zu übertragen und eine Kavitation im Inneren des adipösen Gewebes herbeizuführen, wobei die genannte Sonde (30) wenigstens eine von der genannten Sonde (30) und der Umgebung der Spitze (31) abhängige Resonanzfrequenz aufweist, ein Kontrollsystem (3), das geeignet ist, die Resonanzfrequenz der in die Umgebung eingeführten genannten Sonde (30) zu überprüfen und die Emissionsfrequenz des elektrischen Stroms zu regulieren, um so Frequenzen an die genannte Sonde (30) zu senden, die sich vorrangig nahe an der Resonanzfrequenz der genannten Sonde (30) befinden, wenn die genannte Spitze in bestimmte zu behandelnde Umgebungen eingeführt ist, um so Kavitation nur herbeizuführen, wenn die genannte Spitze (31) in die genannten bestimmten Umgebungen eingeführt ist, wobei das genannte Kontrollsystem (3) geeignet ist, erhebliche Schwankungen der Resonanzfrequenz der genannten Sonde (30) zu signalisieren und so Beschädigungen der nicht adipösen Gewebe zu vermeiden, indem in diesen Fällen die Emission der Frequenz des elektrischen Stroms nicht reguliert wird.

2. Vorrichtung nach Anspruch 1, die geeignet ist, Kavitation nur herbeizuführen, wenn die genannte Spitze (31) in Wasser oder ein Medium ähnlicher Dichte eingetaucht ist.

3. Vorrichtung nach Anspruch 2, bei der die genannte, in Wasser getauchte Sonde (30) eine Resonanzfrequenz von ca. 20 kHz aufweist.

4. Vorrichtung nach Anspruch 2 oder 3, bei der die genannte, in Wasser getauchte Sonde (30) eine Resonanzfrequenz von ca. 40 kHz aufweist.

5. Vorrichtung nach einer oder mehreren der vorangegangenen Ansprüche, bei der die genannte Sonde ein Handstück (30a), das an die genannte Spitze (31) angeschlossen werden kann, und eine Vielzahl von piezoelektrischen Elementen (32) umfasst, die an Führungselemente (34) angeschlossen sind und zwei Sperrkörper (35) und (36), die geeignet sind, die genannten piezoelektrischen Elemente (32) mittels entsprechender Klemmelemente (37) zusammenzuklappen.

6. Vorrichtung nach einer oder mehreren der vorangegangenen Ansprüche, bei der die genannte Spitze (31) aus einer Nadel mit einer Länge zwischen 10 cm und 40 cm und einem Durchmesser zwischen 0,5 mm und 5 mm besteht.

7. Vorrichtung nach einer oder mehreren der vorangegangenen Ansprüche, bei der die genannte Spitze (31) aus Titan Grade 5 besteht.

8. Vorrichtung nach einer oder mehreren der vorangegangenen Ansprüche, bei der die genannte Spitze (31) aus einer langen Spitze (31 a) bestehend aus einer Nadel mit einer Länge zwischen 10 cm und 40 cm und einem Durchmesser zwischen 0,5 mm und 5 mm besteht.

9. Vorrichtung nach einer oder mehreren der vorangegangenen Ansprüche, bei der die genannte Spitze (31) aus einer feinen Spitze (40) mit einem rohrförmigen Abschnitt (42) mit einer Länge zwischen 7 cm und 10 cm und einem Durchmesser ihres Endstücks (41) zwischen 1,5 mm und 2,5 mm besteht.

10. Vorrichtung nach Anspruch 9, bei der der genannte rohrförmige Abschnitt (42) mindestens einen ersten und einen zweiten zylindrischen Abschnitt (42a, 42b) mit unterschiedlichen Durchmessern umfasst.

11. Vorrichtung nach Anspruch 10, bei dem der genannte erste zylindrische Abschnitt (42a) das genannte Endstück (41) umfasst und eine Länge zwischen 3 cm und 6 cm aufweist und einen Durchmesser, der dem Durchmesser des genannten Endstücks (41) entspricht.

12. Vorrichtung nach Anspruch 10 oder 11, bei dem der genannte zweite zylindrische Abschnitt (42b) die Basis der genannten feinen Spitze (40) umfasst und eine Länge zwischen 2 cm und 4 cm und einen Durchmesser zwischen 2,5 mm und 3,5 mm aufweist.

13. Vorrichtung nach einem oder mehreren der Ansprüche 10-12, bei der der rohrförmige Abschnitt (42) einen dritten zylindrischen Abschnitt (42c) zwischen dem genannten ersten und zweiten zylindrischen Abschnitt (42a, 42b) umfasst und eine Länge zwischen 0,5 cm und 1,5 cm und einen Durchmesser zwischen 2,5 mm und 3,5 mm aufweist.

## Revendications

1. Dispositif vibrant pour le traitement de tissus adipeux comprenant : au moins un appareil générateur (2) de courant électrique alternatif, au moins une sonde (30) connectée audit appareil générateur (2) et incluant au moins un élément piézoélectrique (32), apte à transformer ledit courant électrique alternatif en vibrations mécaniques, et au moins un embout (31, 40), apte à être inséré à l'intérieur dudit tissu adipeux, à transmettre lesdites vibrations mécaniques et à entraîner une cavitation à l'intérieur dudit tissu adipeux, ladite sonde (30) présentant au moins une fréquence de résonance variable en fonction de ladite sonde (30) et de la zone environnante audit embout (31), un système de contrôle (3) apte à vérifier la fréquence de résonance de ladite sonde (30) immergée dans la zone environnante et à régler la fréquence d'émission du courant électrique de sorte à envoyer à ladite sonde (30) des fréquences essentiellement proches de la fréquence de résonance de ladite sonde (30) lorsque ledit embout est immergé dans des zones déterminées à traiter, de façon telle à entraîner une cavitation uniquement si ledit embout (31) est immergé dans lesdites zones déterminées, dans lequel ledit système de contrôle (3) est apte à signaler des variations importantes de la fréquence de résonance de ladite sonde (30) et à éviter des endommagements des tissus non adipeux sans régler, dans ces cas, l'émission de fréquence du courant électrique.

2. Dispositif selon la revendication 1, apte à entraîner une cavitation uniquement si ledit embout (31) est immergé dans l'eau ou dans un fluide de densité similaire.

3. Dispositif selon la revendication 2, dans lequel ladite sonde (30) immergée dans l'eau présente une fréquence de résonance proche de 20 kHz.

4. Dispositif selon la revendication 2 ou 3, dans lequel ladite sonde (30) immergée dans l'eau présente une fréquence de résonance proche de 40 kHz.

5. Dispositif selon une ou plusieurs des revendications précédentes dans lequel ladite sonde comprend une poignée (30a) pouvant être raccordée audit embout (31) et comprenant une pluralité d'éléments piézoélectriques (32) reliés à des éléments conducteurs (34) et deux corps de blocage (35) et (36) aptes à fermer en sandwich lesdits éléments piézoélectriques (32) par l'intermédiaire de moyens de serrage spécifiques (37).

6. Dispositif selon une ou plusieurs des revendications précédentes, dans lequel ledit embout (31) est constitué d'une aiguille ayant une longueur comprise entre 10 cm et 40 cm et un diamètre compris entre 0,5 mm et 5 mm.

7. Dispositif selon une ou plusieurs des revendications précédentes, dans lequel ledit embout (31 ) est en titane de degré 5.

8. Dispositif selon une ou plusieurs des revendications précédentes, dans lequel ledit embout (31) est constitué d'un embout long (31 a) constitué d'une aiguille ayant une longueur comprise entre 10 cm et 40 cm et un diamètre compris entre 0,5 mm et 5 mm.

9. Dispositif selon une ou plusieurs des revendications précédentes, dans lequel ledit embout (31) est constitué d'un embout fin (40) comprenant une partie tubulaire (42) ayant une longueur comprise entre 7 cm et 10 cm et un diamètre de sa partie terminale (41), compris entre 1,5 mm et 2,5 mm.

10. Dispositif selon la revendication 9, dans lequel ladite partie tubulaire (42) comprend au moins une première et une deuxième partie cylindrique (42a, 42b) ayant des diamètres différents.

11. Dispositif selon la revendication 10, dans lequel ladite première partie cylindrique (42a) comprenant ladite partie terminale (41) et ayant une longueur comprise entre 3 cm et 6 cm et un diamètre équivalent au diamètre de ladite partie terminale (41).

12. Dispositif selon la revendication 10 ou 11, dans lequel ladite deuxième partie cylindrique (42b) comprenant la base dudit embout fin (40) a une longueur comprise entre 2 cm et 4 cm et un diamètre compris entre 2,5 mm et 3,5 mm.

13. Dispositif selon une ou plusieurs des revendications 10-12, dans lequel ladite partie tubulaire (42) comprend une troisième partie cylindrique (42c), interposée entre ladite première et ladite deuxième partie cylindrique (42a, 42b) et ayant une longueur comprise entre 0,5 cm et 1,5 cm et un diamètre compris entre 2,5 mm et 3,5 mm.
